# EUROPEAN PATENT APPLICATION

(11) **EP 4 660 109 A1**
(43) Date of publication of application: **10.12.2025**
(21) Application number: 25174378.7
(22) Date of filing: 06.05.2025
(51) Int. Cl.: B65G 53/46, F16K 31/04

(54) **HYGIENIC ROTARY VALVE**

(30) Priority: 04.06.2024 TW 113121316
(71) Applicant: Chen, Kao-Sung, Tainan City 702014 (TW)
(72) Inventor: Chen, Kao-Sung, Tainan City 702014 (TW)
(74) Representative: Jannig & Repkow Patentanwälte PartG mbB

(57) **Abstract**

A hygienic rotary valve is designed to meet the hygiene application requirements of handling powdered and granular materials in the food industry. The hygienic rotary valve is a material conveying device installed beneath a powdered and granular material silo, and enhances operational capabilities of the rotary valve by improving the flow rate of powdered and granular materials; providing viewing windows to view the internal state; simplifying the disassembly and assembly procedures (Easy Access) to reduce cleaning process time; implementing Clean-In-Place (CIP) and Steam-In-Place (SIP) processes. The hygienic rotary valve includes a valve body, guide rails, a gear motor, a side cover, a transmission shaft, an end cover, a rotor, a front door, and a device for blowing off adhered powdered and granular materials. In a dust-filled working environment, air purge protection is provided to prevent powdered and granular materials from invading the power transmission area.

## Description

### FIELD OF THE INVENTION

The present invention relates to a hygienic rotary valve designed to meet the hygienic application needs of the food industry for handling powdered and granular materials.

### BACKGROUND OF THE INVENTION

In powdered and granular materials factories, silos are the primary storage equipment, and rotary valves are the main devices for controlling the transportation and flow rate of these materials. The rotary valve is installed at the bottom of the silo. Its structure consists of a hollow valve body with an inlet at the top, an outlet at the bottom, a door on each of two sides of the silo, and a side cover sealing the valve body of the silo. Inside the rotary valve, there is a rotor. A motor seat is connected to a gear motor and located on the outside of the valve body. The gear motor drives the rotor within the valve body in a controlled manner through a transmission shaft, effectively discharging the powdered and granular materials from the silo. The rotary valve is one of the main production equipment in powdered and granular material factories.

The importance of food safety cannot be overstated. The raw materials for food are organic substances, and some materials begin to oxidize and spoil within about three to four hours under suitable conditions, leading to bacterial growth. During processing, the surfaces of food processing equipment may become contaminated by harmful microorganisms transferred from the powdered and granular materials. According to relevant international food hygiene regulations, food processing plants must select qualified equipment to implement effective cleaning and sterilization processes, which are necessary measures to maintain food hygiene safety.

Most powdered and granular material transportation systems use pneumatic conveying, where gas flow creates areas of positive and negative pressure. The rotor of the rotary valve is a moving part, and there are necessary gaps between the rotor and the valve body of the silo. When transporting materials, particles inevitably seep into these gaps, and occasionally, unclean particles are drawn out by the pneumatic force, leading to cross-contamination. To ensure food hygiene and safety, regularly implementing suitable and effective cleaning methods is an important and necessary daily operation for food factories.

The surfaces of food processing equipment are divided into contact surfaces and non-contact surfaces (with the food). Strict standards are set for the smoothness of the contact surfaces. If the contact surface is damaged (e.g., scratched) during operations due to equipment failure or other reasons, the equipment cannot be used for production according to hygiene safety standards. Even if polished and repaired, it is difficult to restore the surface to a uniform smoothness, thus making it unusable for production.

Cleaning and sterilization are applied to the equipment and material contact surfaces, with cleaning methods varying according to the nature of the materials (e.g., oily or sticky products). The preferred cleaning method is dry cleaning. If dry cleaning is insufficient to remove dirt or allergens, wet cleaning must be used. For certain bacteria requiring high-temperature sterilization, steam is the ideal option to achieve thorough sterilization.

To improve cleaning operation efficiency and reduce production downtime, food processing equipment should be designed for easy disassembly and reassembly (Easy Access).

Before starting the cleaning process, the internal transport system needs to be unobstructed so that water, cleaning agents, hot air, or steam can reach all parts of the system. This ensures the effectiveness of Cleaning In Place (CIP) and Steam In Place (SIP). Due to the need for transport functions, the rotor of the rotary valve is placed at the center of the valve body, dividing the entire transport system into inner and outer parts. During cleaning, the rotor blocks the cleaning passage, meaning that the cleaning process cannot proceed without removing the rotor.

During steam sterilization, the transport system is filled with saturated steam to raise the internal temperature to 130°C and maintain this temperature for over half an hour. The process requires a platform to operate and monitor the steam dynamics, adjusting the pressure of the saturated steam to maintain a stable temperature. As steam contacts the silo walls, it continuously generates condensate, which needs to be drained periodically.

Moreover, various factors such as environmental temperature and humidity, the moisture content of materials, and static electricity generated by friction cause materials to adhere to the inner walls of the rotor during transport. This reduces the utilization rate of the chamber space, leading to decreased transport efficiency.

The conventional rotary valve comprises a valve body containing a rotor. The rotor is formed by welding several metal blades into a ring shape around a long shaft, which serves as its transmission shaft. Two ends of the transmission shaft pass through the doors one both sides and the side cover, and are held in place at the center of the doors and side cover by bearings.

The conventional rotary valve only has the basic function of transporting powdered and granular materials and lacks other functionalities. It is difficult and time-consuming to disassemble and assemble, and its cleanability is poor, not meeting the standards and conditions required for food processing equipment.

The conventional rotary valve has a complex transmission structure that requires special tools for disassembly and assembly, making the process laborious and time-consuming. Additionally, its shaft seal is exposed, making this area difficult to clean and dry, and its lubricating oil can potentially contaminate raw materials.

In the conventional rotary valve, a shaft seal is placed between the transmission shaft and the bearings, usually made of elastomer. This shaft seal gradually wears out due to operational weight and friction, causing the rotor to deflect and eventually touch and scratch the valve body, damaging the contact surface. Once the contact surface of the rotary valve is damaged, it no longer meets food processing equipment hygiene and safety standards.

To emphasize the advantage of quick disassembly and assembly, rotary valves with guide rails adopt a loose fit for the transmission mechanism to simplify the process. However, this loose fit mechanism causes the rotor to deflect, leading to the aforementioned issues.

The conventional rotary valve cannot achieve a sealed pressurized state and cannot implement steam sterilization.

The conventional rotary valve lacks auxiliary devices, such as inspection ports and air blow-off powdered and granular materials devices.

The present invention intends to provide a hygienic rotary valve to eliminate the shortcomings mentioned above.

### SUMMARY OF THE INVENTION

This invention is an extension based on the references of US Patent Publication No. 20110025520A1 and European Patent Publication No. 2280203A2, "Sanitary Rotary Valve." The present invention also references standards such as DIN 10516: 2001, EN 1672-2: 2020, DIN EN ISO 14159: 2008, NSF/ANSI 8: 2012, and ASME BPE.

The objective of the present invention is to solve the aforementioned issues by providing a hygienic rotary valve that is highly cleanable and easy to operate. This hygienic rotary valve is a key component in the powdered and granular material conveying systems in the food industry and includes a highly cleanable design (Cleanability) and easy operation (Easy Access).

The present invention relates to a hygienic rotary valve, and comprises a valve body with a top opening connected to a silo, and a bottom opening connected to a conveying pipeline. A front port and a rear port are defined in a front end and a rear end of the valve body respectively. Two guide rails are connected to both sides of the valve body.

A side cover is slidably connected to the guide rails, and the side cover matches and seals the rear port of the valve body. The side cover includes a transmission shaft seat, and includes a power structure and an air purging device. The power structure comprises a gear motor, a motor seat, a coupling, a transmission shaft, bearings, and shaft seals. The transmission shaft passes through the transmission shaft seat and is correspondingly installed inside the valve body.

The transmission shaft has an end corresponding to the coupling, with its middle section slightly tapered to match the center of the inner wall surface of the rotor. The front end of the transmission shaft has a multiple-faced column to correspond to the inner hexagonal slot of the rotor, enabling the rotation of the rotor. Additionally, the front end of the transmission shaft is axially recessed to form a recessed section.

The rotor is shaftless and a hollow inner rotor ring is located at the center of the rotor. The inner wall surface of the inner rotor ring is slightly inclined to correspond to the conical section of the transmission shaft. The inner rotor ring includes an inner polygonal slot to match the multiple-faced column of the transmission shaft. The rotor is radially equipped with multiple blades, forming an equal number of chambers from either 8 or 12 blades. These chambers are semi-open, open on the side facing the front door to receive air jets from it, and closed on the side facing the side cover to restrict the path of falling powdered and granular materials.

The end cover corresponds to and attaches to the recessed area at the front end of the transmission shaft, covering a connection gap between the rotor and the transmission shaft. The end cover uses thread force to tighten the rotor against the transmission shaft and is designed without sharp angles to prevent powdered and granular materials accumulation. The center of the end cover features a hexagonal column corresponding to the socket for tightening or loosening.

The front door covers the front port of the valve body and features a first air nozzle corresponding to the end cover, and at least one second air nozzle corresponding to the chambers of the rotor. The first and second air nozzles are connected to high-pressure air filtration device, which blow high-pressure air to remove powdered and granular materials adhering to the chambers of the rotor. The second air nozzle and its high-pressure air filtration device blow air deep into the chambers of the rotor, while the first air nozzle and the high-pressure air filtration device blow air into the recess of the end cover located at the center of the rotor. This blow-down function helps remove material adhering to the internal surfaces, maintaining the utilization of internal space.

The motor seat includes a coupling for being connected with the gear motor and the transmission shaft.

The gear motor outputs power.

A set of parallel rails supports the gear motor and the side cover. A guard plate is installed to the distal ends of the rails, with warning signs at the front and back. Below the guard plate is an integrated storage rack that can hold tools and fasteners. This set of rails is fixed and does not move, providing a stable and precise sliding platform for the side cover and the gear motor.

In a conveying mode, the rotor is driven to rotate by the transmission shaft powered by the gear motor, used to transport materials. At the same time, the high-pressure air filtration devices blow high-pressure air through the first air nozzle and the second air nozzle to remove powdered and granular materials adhering to the surface of the end cover or the chambers of rotor.

The valve body can be used for dry cleaning mode, wet cleaning mode, and steam sterilization mode.

In the dry cleaning mode, the front door can be opened and the cover plate are removed. The gear motor and the side cover are pushed back along the rails, allowing the rotor to exit through the rear port of the valve body along with the transmission shaft. The tools can be stored on the storage rack. This process does not require disassembling the bearings and shaft seals, and only involves unscrewing the fasteners of the front door and side cover.

In the wet cleaning mode, following the steps of the dry cleaning mode, the cover plates are installed at the front port and the rear port of the valve body to perform wet cleaning of the conveying equipment.

In the steam sterilization mode, the first pressure-resistant door is installed at the front port of the valve body, and the second pressure-resistant door is installed at the rear port. A steam inlet/outlet valve is installed on the first pressure-resistant door. A thermometer and a pressure gauge are installed on the second pressure-resistant door. Additionally, a third pressure-resistant door with a drainage valve is installed at the bottom opening of the valve body, where the drainage valve has a drainage port for the input and discharge of condensate through the steam inlet/outlet valve to perform steam sterilization.

In the wet cleaning mode of the hygienic rotary valve described above, the cover plate seals the rotary valve and can be transparent, functioning as a viewing window. This wet cleaning mode follows the cleaning procedures of food factories, allowing the entire conveying system to be effectively wet cleaned through the rotary valve.

In the steam sterilization mode of the hygienic rotary valve described above, the first pressure-resistant door is installed at the front port of the valve body, and the second pressure-resistant door is installed at the rear port. The third pressure-resistant door is installed at the bottom opening of the valve body, turning the hygienic rotary valve into a pressure-resistant container. The second pressure-resistant door is equipped with a thermometer and a pressure gauge for monitoring. The third pressure-resistant door at the bottom of the valve body has a drainage valve with a drainage port for discharging condensate.

The steam sterilization cleaning mode follows the cleaning procedures of food factories. Through the rotary valve, the entire conveying system can be effectively steam sterilized.

Preferably, multiple bearings and at least one shaft seal are installed between the transmission shaft seat of the side cover and the transmission shaft. The bearings support the rotor, while the shaft seal prevents the intrusion of powdered and granular materials.

Preferably, a plastic sealing ring is installed between the end of the side cover and the conical section of the transmission shaft. This plastic sealing ring blocks powdered and granular materials from entering the gap between the side cover and the transmission shaft.

Preferably, the side cover is further equipped with two orifices configured as air injection pipelines. These are connected to the high-pressure air filtration devices, which introduce high-pressure air to evenly press the plastic sealing ring. This forces the plastic sealing ring to seal the gap between the end of the side cover and the conical section of the transmission shaft. This setup functions as an ingress protection device. High-pressure air is injected into the gap between the side cover and the transmission shaft through these two orifices, with the plastic sealing ring being pressed by the high-pressure air to block the gap. The high-pressure air is injected in two streams to distribute the pressure evenly, effectively preventing dust from intruding.

Preferably, the side cover further includes a protruding ring portion corresponding to the rear port of the valve body, and the outer circumferential surface of the protruding ring portion is further provided with an elastic sealing element, which elastically seals between the rear port of the valve body and the side cover. The elastic sealing element can be a rubber sealing ring, thereby ensuring a complete seal when the side cover is assembled to the valve body. The side cover has a recessed annular groove at the position corresponding to the rotor, with an elastic shaft seal and a wear-resistant shaft seal sequentially arranged from inside to outside in the annular groove, and the wear-resistant shaft seal is in contact with the rotor. The elastic shaft seal can be a rubber shaft seal with compressible elastic properties. Combined with the wear-resistant shaft seal, which can be made of wear-resistant PTFE (polytetrafluoroethylene), the rubber shaft seal presses the wear-resistant shaft seal tightly against the rotor. This allows the rotor to be driven and operated stably while also being pressed by the elastic rubber shaft seal to provide a seal that prevents powdered and granular materials from intruding between the side cover and the rotor.

Preferably, the valve body is further provided with multiple legs, which protrude from the bottom end of the valve body to support the valve body.

Preferably, the front door is additionally equipped with a bolt. The valve body has lugs on its side, and the front door is connected to the lugs via the bolt, allowing the front door to rotate and switch at the front port of the valve body.

Preferably, at the front end of the conical section, there is a multiple-faced column. The rotor is designed with an inner hexagonal slot corresponding to the multiple-faced column, allowing the transmission shaft to engage the rotor through the multiple-faced column and the inner hexagonal slot.

Preferably, it also includes a tool which has a socket at one end, corresponding to the hexagonal column at the center of the end cover, used to rotate the end cover. At the other end of the tool is a pry bar, which is equipped with a hook corresponding to the rotor. When disconnecting the rotor from the transmission shaft, the hook can be engaged with the edge of the rotor to lever the rotor.

Preferably, the front door is equipped with at least one viewing window which is fitted with a transparent panel, allowing visibility into the interior of the valve body during operation.

The primary advantages and effects of the present invention are as follows:
1. Enhanced Carrying and Conveying Efficiency: By configuring the blades of the rotor, chambers can be formed, improving the carrying and conveying efficiency of powdered and granular materials.
2. Easy Disassembly and Assembly (Easy Access): The present invention allows for easy disassembly and assembly through the hinged front door and the sliding side cover and guide rails. When the side cover is opened, the rotor can be pulled out parallel from the valve body to prevent scratches on the inner wall. Tools like a socket can then be used to remove the end cover, and a pry bar can loosen the rotor for removal from the transmission shaft, facilitating cleaning. Conversely, reassembly only requires simple reconnection of the transmission shaft and end cover, after which the rotor and side cover can be pushed back into the valve body to complete the assembly.
3. Adaptable Cleaning Modes: The present invention can be configured for dry cleaning, wet cleaning, and steam sterilization modes according to various cleaning requirements. This includes not only general wiping but also Clean-In-Place (CIP) and Steam-In-Place (SIP) processes. In the wet cleaning mode, two cover plates (e.g., transparent plastic plates) can be installed at the front and rear ports of the valve body, making the rotary valve enclosed again for wet cleaning. These transparent plastic plates also serve as inspection windows, allowing the internal state during wet cleaning to be viewed. In the steam sterilization mode, the first and second pressure-resistant doors are installed at the front and rear ports, respectively. The first pressure-resistant door is equipped with a steam inlet/outlet valve controlled remotely, while the second pressure-resistant door has a thermometer and pressure gauge for monitoring. In this mode, the entire interior becomes a pressure vessel meeting the requirements of pressure vessel directives, thus ensuring safety standards.
4. High-Pressure Air Blowing for Material Detachment and Intrusion Protection: The first set of high-pressure air blowers removes materials adhering to the rotor and the inner wall of the recess at the end of the transmission shaft. Additionally, the second air purging device uses high-pressure air to press the plastic sealing ring, forcing it to seal effectively between the end of the side cover and the conical column of the transmission shaft, forming an intrusion protection device. This effectively prevents powdered and granular materials from invading the power transmission area, reducing component wear and affecting transmission. This design provides high stability and reduces wear, significantly extending the lifespan of the invention.

The present invention will become more obvious from the following description when taken in connection with the accompanying drawings which show, for purposes of illustration only, a preferred embodiment in accordance with the present invention.

### BRIEF DESCRIPTION OF THE DRAWINGS

Fig. 1 is a perspective view of the hygienic rotary valve of the present invention;
Fig. 2 is perspective view of the hygienic rotary valve of the present invention from another angle;
Fig. 3 is an exploded view of the hygienic rotary valve of the present invention;
Fig. 4 is a perspective view of the hygienic rotary valve of the present invention at the front door;
Fig. 5 is a cross sectional view, taken along line A-A of Fig. 1;
Fig. 6 is a cross sectional view, taken along line B-B of Fig. 1;
Fig. 7 is a perspective view showing the rotation of the end cover with a socket using a pry bar;
Fig. 8 is a perspective view showing an action on the rotor with a hook using the pry bar;
Fig. 9 is a perspective view to show that the front door is opened, the side cover is pushed back, and the rotor removed;
Fig. 10 is another perspective view of Fig. 9 from a different angle;
Fig. 11 is a perspective view of the present invention in a wet cleaning mode.
Fig. 12 is another perspective view of Fig. 11 from a different angle;
Fig. 13 is a cross sectional view of the present invention in the wet cleaning mode;
Fig. 14 is a perspective view of the present invention in a steam sterilization mode;
Fig. 15 is another perspective view of Fig. 14 from a different angle, and
Fig. 16 is a cross sectional view of the present invention in the steam sterilization mode.

### DETAILED DESCRIPTION OF THE PREFERRED EMBODIMENT

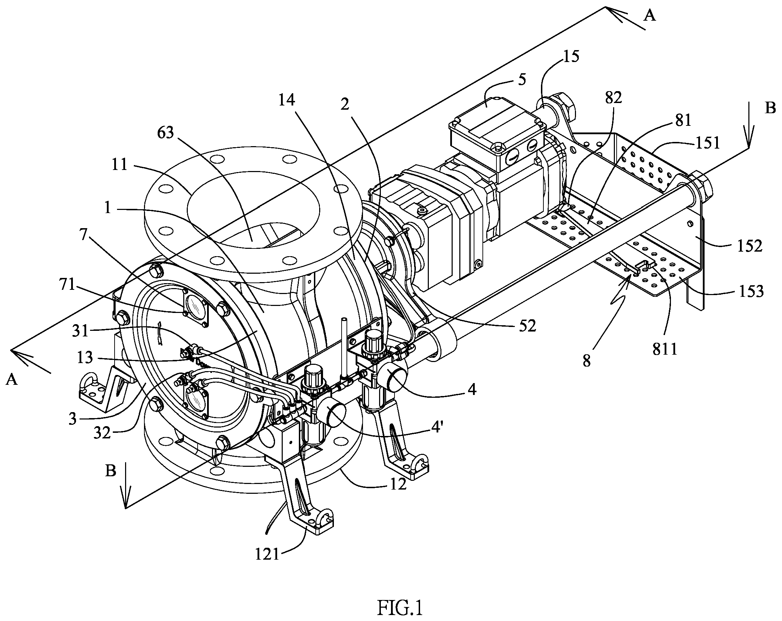

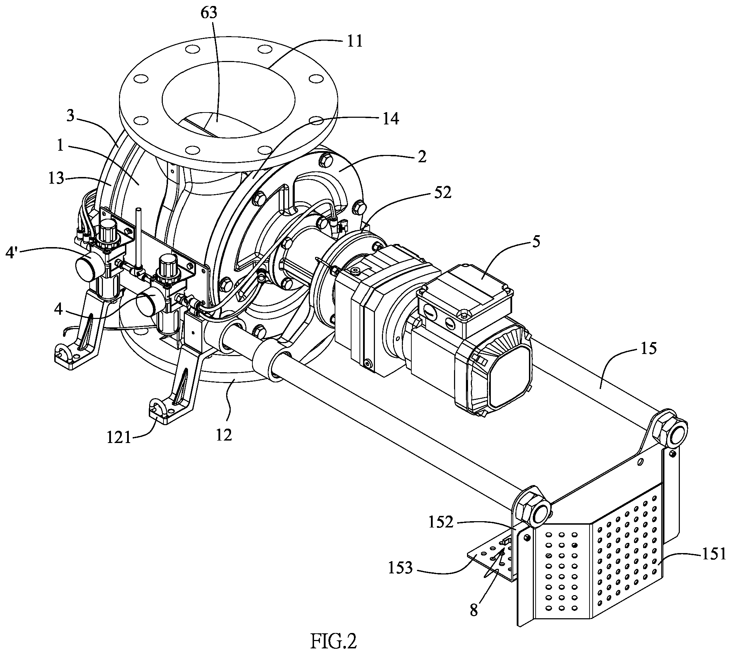

Referring to Figs. 1 to 6, the hygienic rotary valve of the present invention comprises a valve body 1 with a top opening 11 and a bottom opening 12. A front port 13 and a rear port 14 are defined in a front end and a rear end of the valve body 1 respectively. Two guide rails 15are respectively connected to both sides of the valve body 1. The guide rails 15 are axially parallel to the rear port 14 of the valve body 1. The guide rails 15 do not move and serve as a safety and labor-saving device. During disassembly and assembly, the guide rails 15 provide a protective platform. The operation method of this will be described in detail later.

A side cover 2 is slidably connected to the guide rails 15, and the side cover 2 matches and seals the rear port 14 of the valve body 1. The side cover 2 includes a transmission shaft seat 21.

A front door 3 seals the front port 13 of the valve body 1, and the front door 3 includes a first air nozzle 31 located corresponding to the end cover 65. At least one second air nozzle 32 is located corresponding to an outer surface of the rotor inner ring 61. The first air nozzle 31 and the second air nozzle 32 are connected to a high-pressure air filtration devices 4, 4' respectively.

A gear motor 5 is connected to a transmission shaft 51. The transmission shaft 51 passes through the transmission shaft seat 21 and is correspondingly installed inside the valve body 1. At the front end of the transmission shaft 51, there is a conically shaped conical section 511. Additionally, the front end of the transmission shaft 51 has a recessed section 512.

A rotor 6 is designed to be shaftless, with a hollow structure at its center to allow the transmission shaft 51 to pass through. This rotor 6 includes a rotor inner ring 61, and multiple blades 63 are radially installed on the rotor inner ring 61. The rotor 6 is primarily categorized into open and closed types, with this invention using a semi-open rotor 6. The rotor 6 is positioned at the center of the valve body 1, and consists of eight or twelve blades 63 forming an equal number of chambers 631. The volume of these chambers 631 and the rotation speed of the rotor 6 determine the powdered and granular materials conveying rate. The rotor inner ring 61 has a shaft hole 611 with an inner wall that is slightly inclined to match the tapered column 511 of the transmission shaft 51.

Accordingly, in the drive mechanism of the present invention, the side cover 2 serves as the central position for the entire power structure, acting as the fulcrum for overall force. For the rotational transmission of the transmission shaft 51, the side cover 2 acts as a supporting fixture without contacting the rotor 6 and the transmission shaft 51. The side cover 2 contains four bearings 22, arranged in pairs to support the force points of the transmission shaft 51 and the center of gravity of the rotor 6. The load strength requirements of the transmission shaft 51 and the bearing 22 positions are calculated to ensure that the single-point cantilever can support the weight of the rotor 6 and the conveyed powder. Additionally, at least one shaft seal 25 which is placed between the transmission shaft seat 21 of the side cover 2 and the drive shaft 51, and located before and after the transmission shaft 51. The at least one shaft seal 25 is located at the outer periphery of the bearing 22 in an axial direction of the bearing 22 so as to prevent powdered and granular materials infiltration.

As for the gear motor 5 driving configuration with the drive shaft 51, it is achieved by installing a motor seat 52. The motor seat 52 is in contact with the side cover 2, and a coupling 53 is centrally installed on the motor seat 52. This coupling 53 connects the gear motor 5 to the drive shaft 51, and the coupling allows the absorption of vibrations, ensuring smooth and stable operation, which can extend the lifespan of the bearings 22.

The distal end of the transmission shaft 51 that contacts the rotor 6 is a conical section 511, fitting tightly with the inner wall of the rotor 6, ensuring that the rotor 6 remains aligned with the central axis during operation. The transmission between them is achieved through a multi-faced column 513 at the front end of the conical section 511, fitting into a corresponding inner hexagonal slot 64 on the rotor 6. This tight fit between the multi-faced column 513 and the inner hexagonal slot 64 ensures seamless transmission with no gaps or delays. The conical column 511 fits snugly with the inner wall of the rotor 6, and the rear section of the transmission shaft 51, which is straight, passes through the bearings 22 and shaft seals 25 in the transmission shaft seat 21 of the side cover 2. The rear end of the conical column 511 connects to the coupling 53 in the motor mount 52, enabling the gear motor 5 to drive the rotor 6. This entire connection is tightly fitted with no gaps, preventing deflection during rotor 6 rotation.

An end cover 65 is correspondingly attached to the recessed section 512 at the front end of the transmission shaft 51, covering the connection area between the rotor 6 and the transmission shaft 51. The front end of the drive shaft 51 has threads, and the end cover 65 tightens the rotor 6 against the transmission shaft 51 through the transverse force of the threads. The end cover 65 features a recess 651 and a polygonal prism 652, allowing the end cover 65 to be locked onto the front end of the transmission shaft 51 by rotating the polygonal prism 652. The overall design of the end cover 65 is free of sharp angles, making it easy to clean.

For protection of a gap between the transmission shaft 51 and the side cover 2, a plastic sealing ring 23 is located between a distal end of the side cover 2 and the conical section 511 of the transmission shaft 51 to block powdered and granular materials from entering the gap between the side cover 2 and the transmission shaft 51. The material and the volume of the plastic sealing ring 23 are chosen and calculated to bear the heat due to friction.

The valve body 1 includes two lugs 131, and the front door 3 is mounted onto the lugs 131 via bolts 33 so that the front door 3 of the valve body 1 is rotated to be open and closed 180 degrees at the front port 13. To ensure smoother operation of the front door 3, this design provides a fixed placement position for the front door 3. This allows the front door 3 to remain on the valve body 1 when not in production, without needing to be placed elsewhere, thereby enhancing operational convenience.

To facilitate the observation of the internal workings of the valve body 1 and the rotor 6, the front door 3 is equipped with at least one viewing window 7. The at least one viewing window 7 has a transparent panel 71, which can be made of transparent pressure-resistant plastic, serving as an inspection port for real-time observation of the internal conditions of the valve body 1.

Moreover, since the present invention has a certain weight, in order to ensure that the valve body 1 can be supported on a flat surface under any circumstances, preferably, the valve body 1 is further provided with multiple legs 121. In one embodiment, these legs 121 may be installed at the sides of the valve body 1, and can be integrated or separately assembled. The legs 121 extend downward and protrude from the bottom end of the valve body 1, thereby providing support for the valve body 1 as previously mentioned.

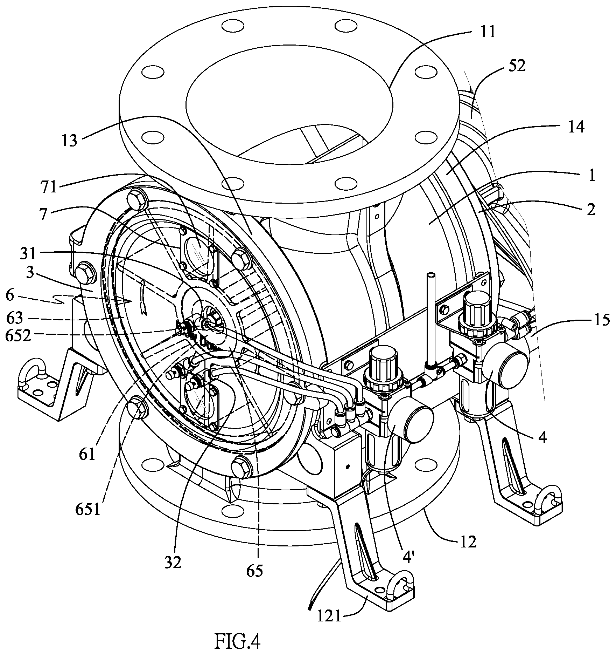

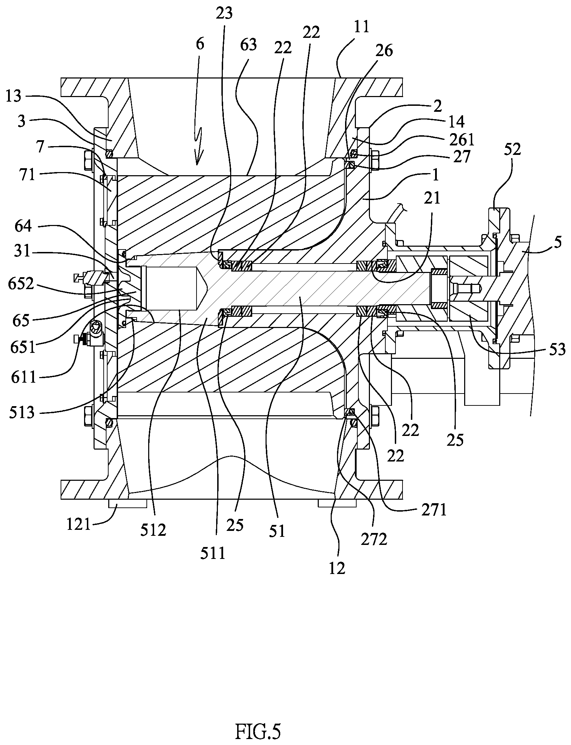

In the conveying mode, as shown in Figs. 4 and 5, the rotor 6 is driven by the gear motor 5 and the coupling 53, which transmit power to the transmission shaft 51, causing the rotor 6 to rotate. As previously described, the chambers 631 of the rotor 6 hold the powder, which is conveyed by the blades 63. During this conveying process, powdered and granular materials may adhere to or accumulate on the rotor 6 or the end cover 65 due to static electricity or other factors. To address this, the present invention utilizes high-pressure air filtering devices 4 and 4' connected by pipelines, and the systems of the two high-pressure air filtering devices 4 and 4' are not interconnected and have different pressures. The high-pressure air filtering device 4 is connected to the first air nozzle 31, which allows it to blow directly into the recess 651 of the end cover 65 via the first air nozzle 31. The high-pressure air stream blows off any powdered and granular materials that may have adhered to the end cover 65, serving as a protective device. The high-pressure air filtering device 4' is connected to the second air nozzle 32. The configuration of the first and two second air nozzles 32 allows high-pressure air to be blown into the deep chambers of the rotor 6, blowing off any powdered and granular materials adhering to the rotor 6. This function is known as blow down, effectively removing the powdered and granular materials from the inner walls of the rotor 6.

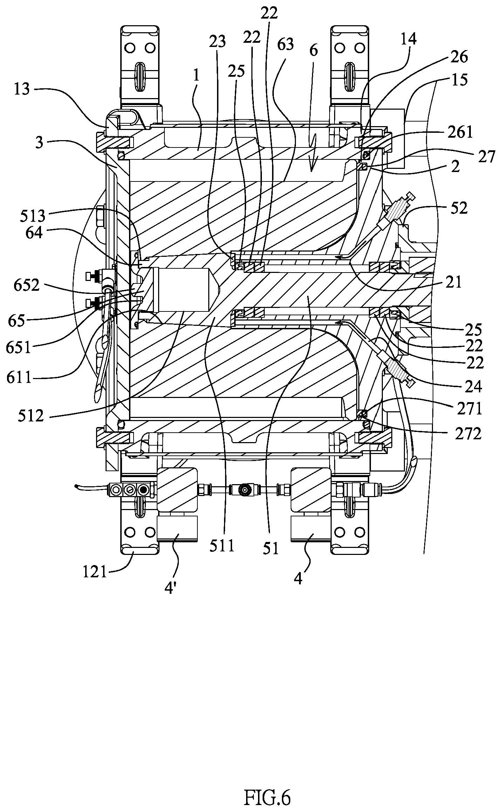

For the protection of the gap between the transmission shaft 51 and the side cover 2, in addition to the previously mentioned plastic sealing ring 23, as shown in Fig. 6, the side cover 2 can be equipped with two orifices 24. These orifices 24 are configured as air injection channels, serving as an ingress protection device. They are connected to the aforementioned high-pressure air filtering devices 4 and 4', used to input high-pressure air to the position where the plastic sealing ring 23 is located. This uniformly presses the plastic sealing ring 23, ensuring it tightly seals the gap between the end of the side cover 2 and the conical section 511 of the transmission shaft 51. A high-pressure zone is thus formed in the gap between the transmission shaft 51 and the side cover 2, providing high-pressure isolation protection to effectively prevent dust from intruding.

Regarding the sealing arrangement between the valve body 1, the side cover 2, and the rotor 6, to enhance the overall safety sealing considerations of the present invention, and to prevent powdered and granular materials from invading between the side cover 2 and the transmission shaft 51 when the high-pressure air filtration devices 4, 4' fail due to power outages, preferably, the side cover 2 can be provided with a protruding ring portion 26 corresponding to the rear port 14 of the valve body 1. The outer circumferential surface of this protruding ring portion 26 is further equipped with an elastic sealing element 261, which can be a rubber sealing ring, to elastically seal between the rear port 14 of the valve body 1 and the side cover 2. This ensures a complete seal when the side cover 2 is assembled to the valve body 1. For protection between the side cover 2 and transmission shaft 51, the side cover 2 has a recessed annular groove 27 at the position corresponding to the rotor 6, with an elastic shaft seal 271 and a wear-resistant shaft seal 272 sequentially arranged from inside to outside in the annular groove 27. The wear-resistant shaft seal 272 is in contact with the rotor 6. It can be understood that the side cover 2 is a fixed component, while the rotor 6 is a movable component that can rotate when driven. To allow the rotor 6 to maintain a seal with the side cover 2 while still rotating stably, the elastic shaft seal 271, with its compressible elastic properties, combined with the wear-resistant shaft seal 272, enables the elastic shaft seal 271 to elastically press the wear-resistant shaft seal 272 and tightly against the rotor 6. This allows the rotor to be driven and operate stably while also being pressed by the elastic shaft seal 271 to provide a seal that prevents powdered and granular materials from intruding into the gap between the side cover 2 and the rotor 6. In one embodiment, the elastic shaft seal 271 can be a rubber shaft seal, and the wear-resistant shaft seal 272 can be a PTFE (polytetrafluoroethylene) shaft seal with wear-resistant characteristics, though this configuration is only exemplary and not limiting.

As previously mentioned, food materials are organic substances, some of which begin to oxidize or breed bacteria within about three to four hours under suitable conditions, leading to spoilage and foul odor. During the production process, the equipment surfaces might become contaminated by harmful microorganisms transferred from the powders. According to international standards on food hygiene and safety, food production equipment must undergo regular and effective cleaning processes to maintain hygiene and safety. Ensuring the hygiene and safety of food requires appropriate and effective cleaning, which is also an essential daily operation in food production factories. This invention can be converted into a dry cleaning mode, a wet cleaning mode, and a steam sterilization mode to accommodate various cleaning methods.

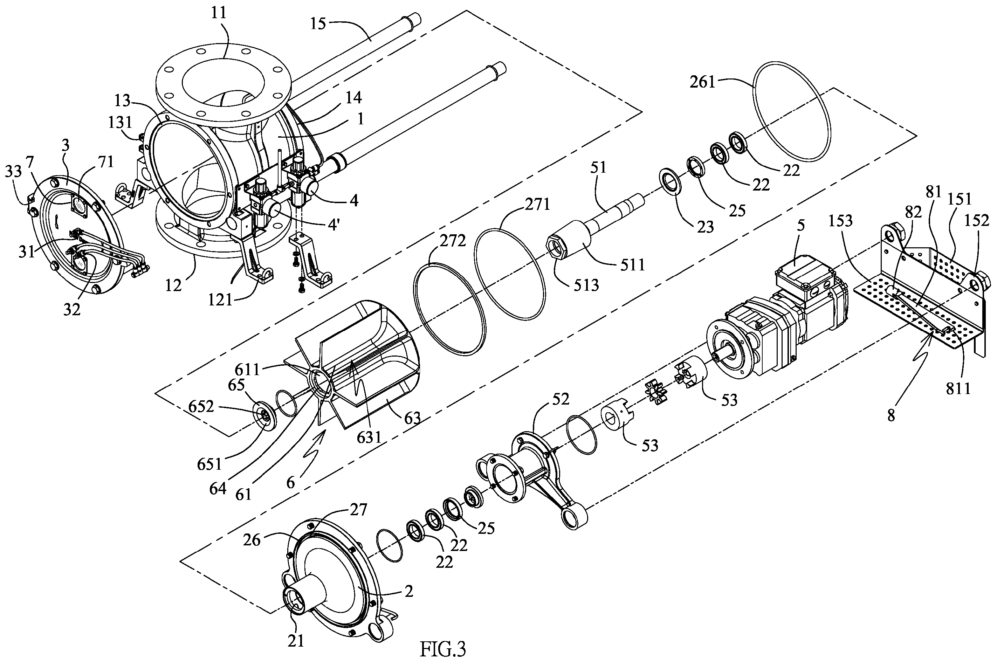

First, before cleaning, the production process in conveying mode should be stopped. Since the front door 3 and the side cover 2 are closed to the valve body 1 with multiple screws, in the dry cleaning mode, as shown in Figs. 3, 7 to 10, the connecting pipes of the high-pressure air filtering devices 4 and 4' should be removed first. After removing the aforementioned screws, the front part can be accessed by opening the front door 3 and rotating it 180 degrees, as previously described, allowing it to remain attached to the valve body 1. This enables the user to dry clean and wipe it directly. For the rear part, the gear motor 5, the side cover 2, and the rotor 6 assembly can be pulled out, with the rotor 6 being withdrawn along with the transmission shaft 51 from the rear port 14 of the valve body 1. As previously mentioned, the guide rails 15 are parallelly arranged along both sides of the rotor inner ring 61 of the valve body 1. The guide rails 15 ensure that the rotor 6 stays centered in the valve body 1 during movement without touching the valve body 1, thus preventing damage to its surface. The inner wall of the valve body 1 has strict smoothness requirements; any scratches would render it non-compliant with food processing equipment hygiene standards, leading to its disposal. The guide rails 15 of the present invention allow the rotor 6 to be precisely and parallelly removed from the valve body 1 without radial movement, completely avoiding contact with the valve body 1, thereby ensuring stability and safety during disassembly and assembly. The distal ends of the guide rails 15 can be equipped with a guard plate 152 for safety markings and to fix the guide rails 15 in place, ensuring it is parallelly aligned. Below the guard plate 152, a storage rack 153 can be set up to temporarily place the necessary tools or disassembled fasteners to prevent loss and improve work efficiency. Warning signs can be placed in front of and behind the guard plate 152 to comply with factory safety regulations.

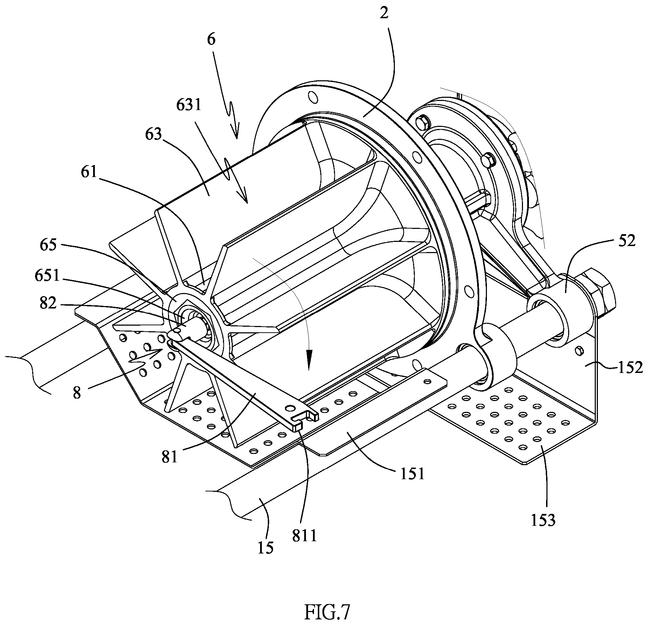

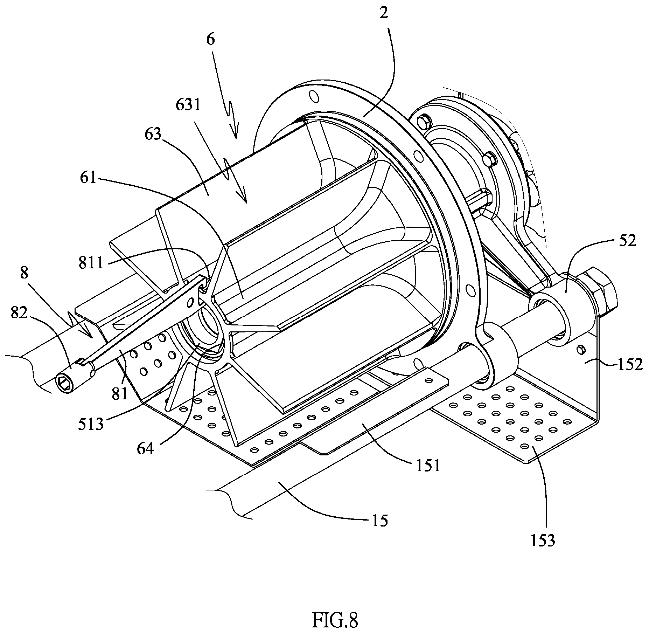

As shown in Figs. 7 and 8, a tool 8 is provided and placed on the valve body 1. One of two ends of the tool 8 is a pry bar 81 which has a hook 811 corresponding to the rotor inner ring 61 of the rotor 6. The hook 811 is engaged with the rotor inner ring 61 of the rotor 6 when removing the rotor 6 from the transmission shaft 51. Another one of the two ends of the tool 8 is a socket 82 for assembling or disassembling the end cover 65. As shown in Fig. 7, the socket 82 can first be fitted onto and used to rotate the polygonal prism 652 of the end cover 65, thereby rotating and removing the end cover 65. Since the rotor 6 is tightly fitted with the conical section 511 and the multi-faced column 513 at the end of the transmission shaft 51, a pry bar 81 with the hook 811 that matches the rotor inner ring 61 can be used to remove the rotor 6 from the transmission shaft 51. The hook 811 is engaged with the rotor inner ring 61, and the rotor 6 can be pried out from the transmission shaft 51. When not in use, the tool 8 can be placed on the aforementioned storage rack 153.

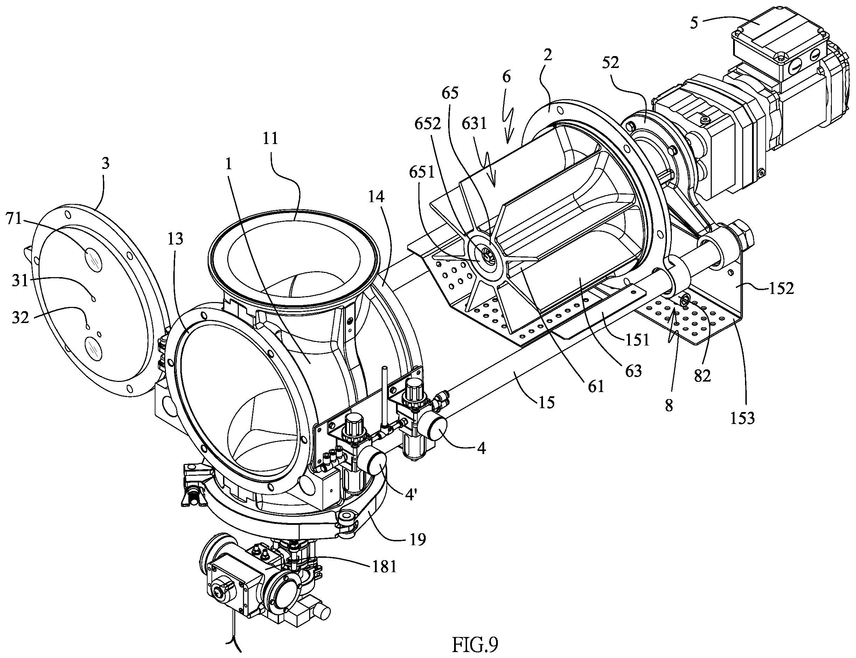

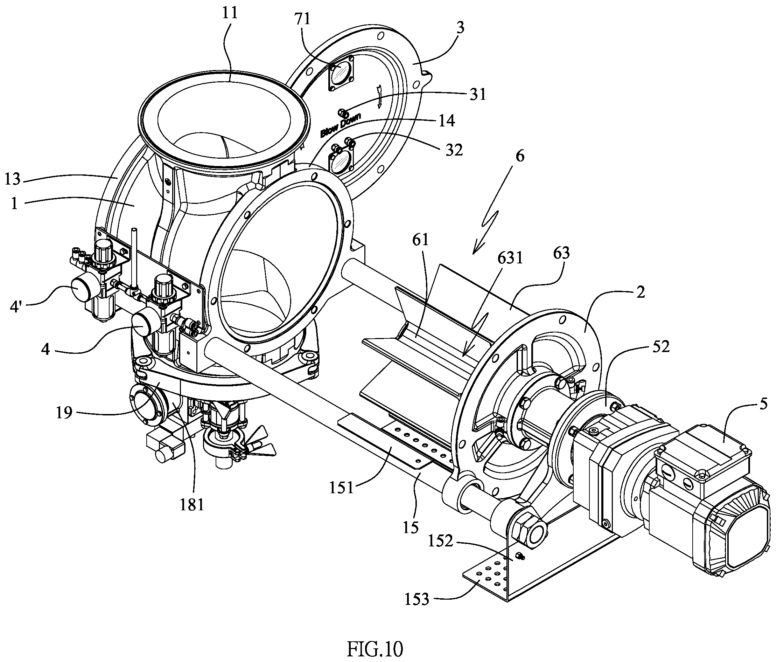

Additionally, since the rotor 6 is quite heavy, before removing it, a tray 151 can be placed on the guide rails 15, as seen in Figures 9 and 10, at a predetermined position corresponding to the removal point of the rotor 6. This allows the rotor 6 to be supported by the tray 151 as it is disengaged from the transmission shaft 51, preventing collisions and enhancing the safety of the rotor 6 removal process. When not in use, the tray 151 can be attached to one end of the storage rack 153, and when needed, it can be removed and placed on the guide rails 15, thereby enhancing convenience during use and allowing the overall invention to be assembled in a modular fashion.

Thus, the entire apparatus can be dry-cleaned according to the factory's operational procedures. This involves dry wiping and cleaning the front door 3, the inner walls of the valve body 1, the transmission shaft 51, the end cover 65, and the rotor 6. After cleaning, reassemble by following the reverse steps, ensuring proper installation and system functionality before resuming production.

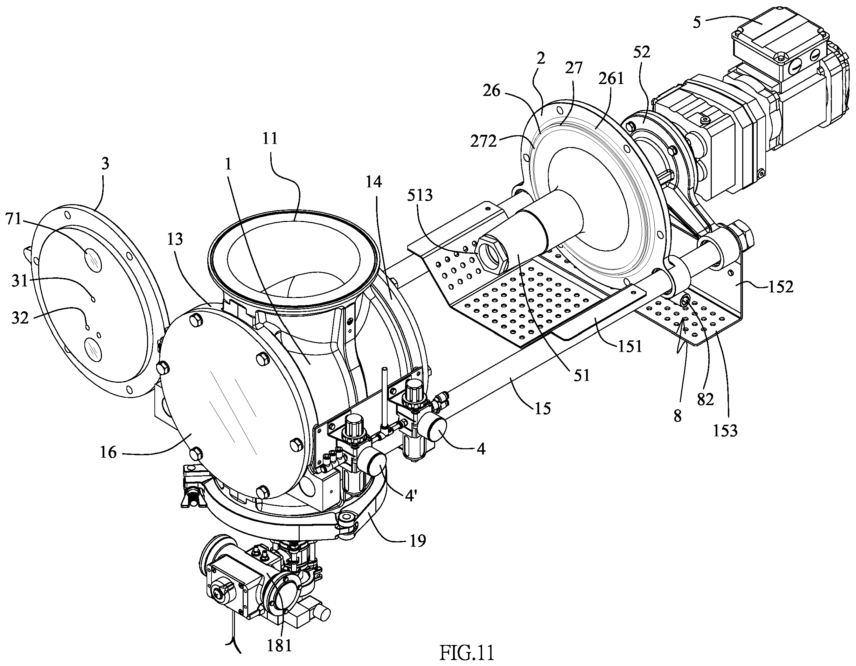

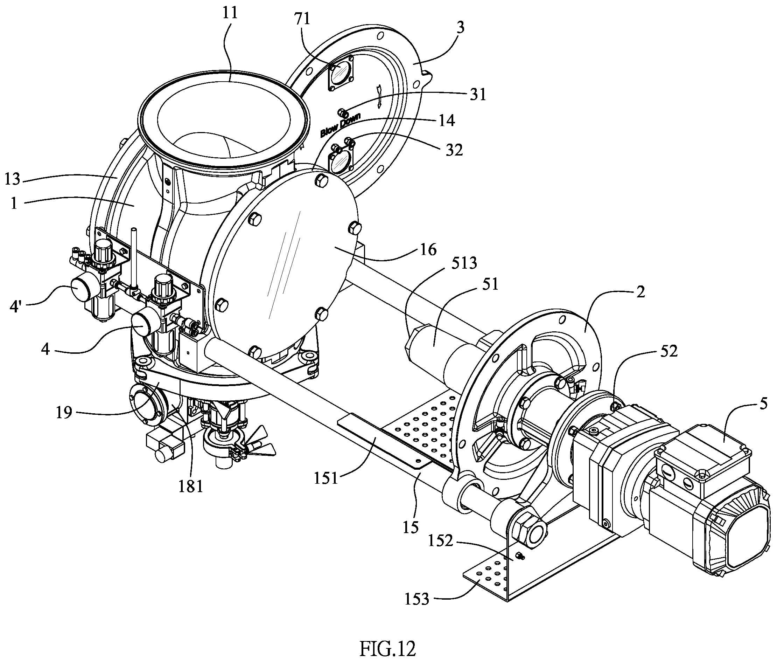

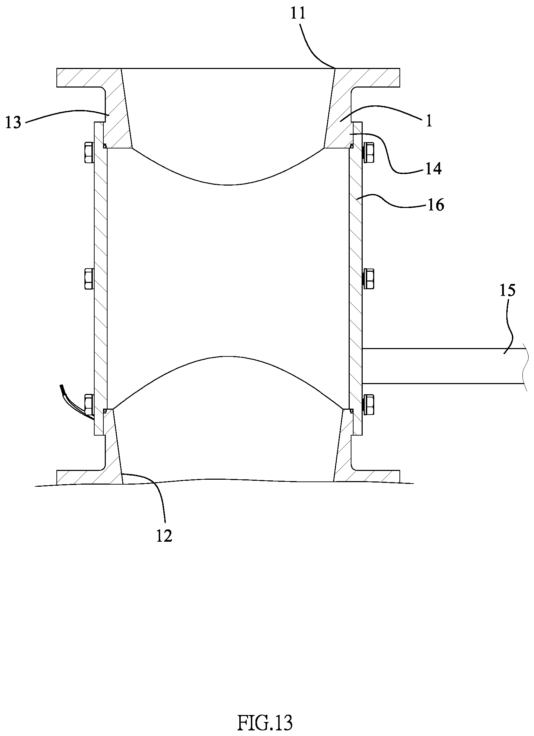

For wet cleaning procedures, as shown in Figs. 11 to 13, the present invention can be configured into a wet cleaning mode. In this mode, while maintaining the disassembled state from the dry cleaning mode, a cover plate 16 is installed at both the front port 13 and the rear port 14 of the valve body 1, allowing wet cleaning through the top opening 11 and bottom opening 12. Preferably, the cover plate 16 is made of transparent plastic, making the valve body 1 sealed again. Wet cleaning can then be performed according to the factory's internal procedures. The cover plate 16 also serves as a window inspection port for monitoring the interior during wet cleaning. The bottom opening 12 of the valve body 1 has a third pressure-resistant door 18, which is equipped with a drainage valve 181, allowing for drainage during wet cleaning. The detailed configuration and explanation will be described in detail later.

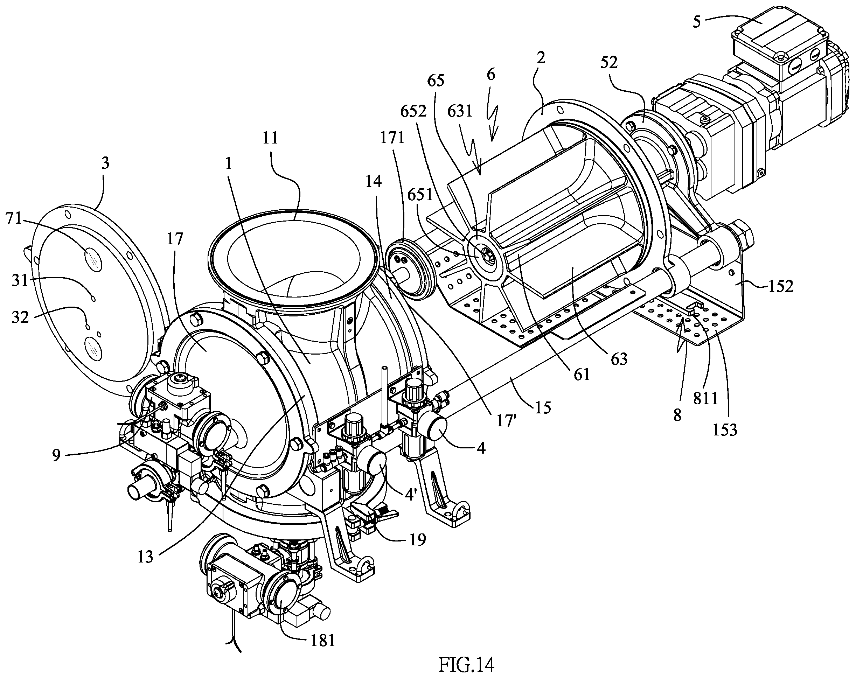

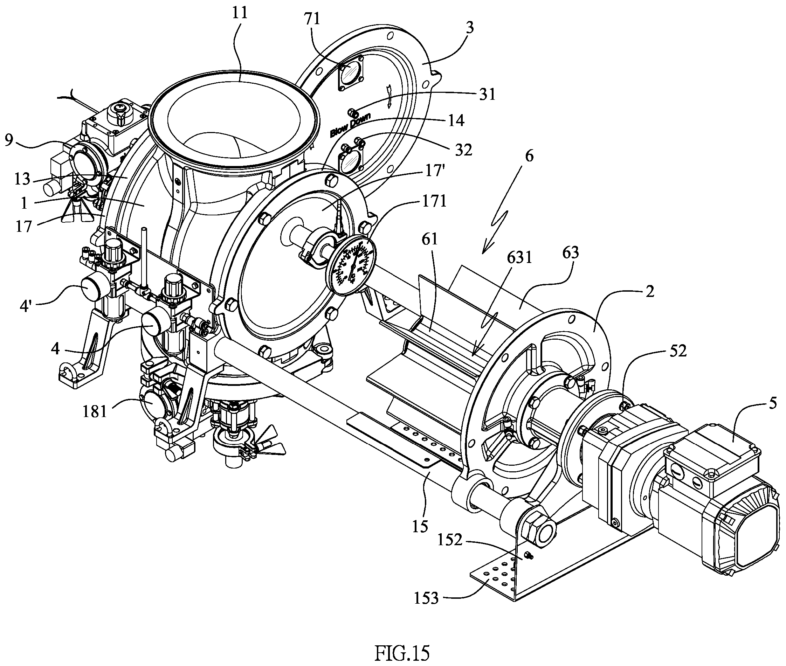

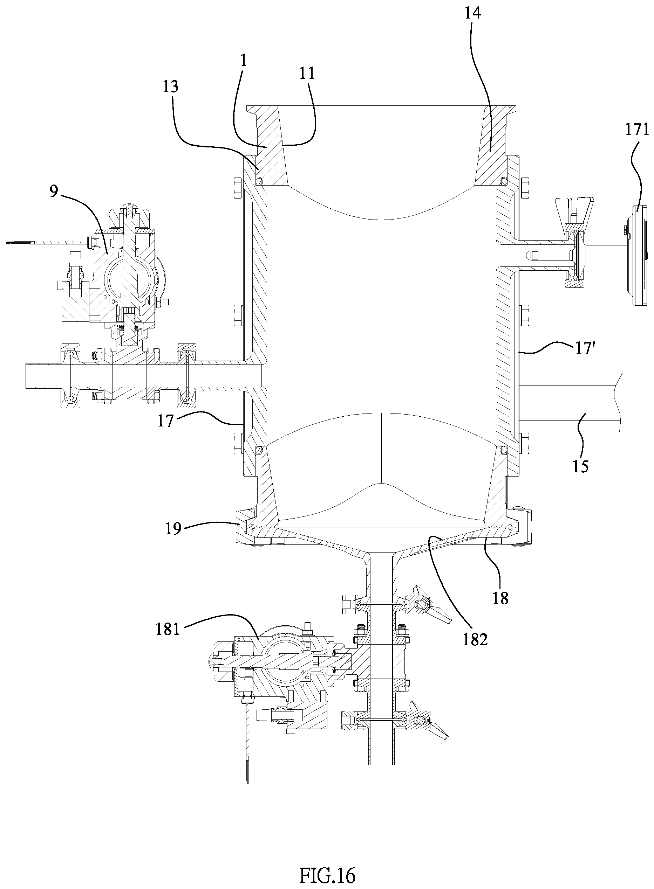

For steam sterilization procedures, after wet cleaning, the apparatus remains disassembled and is reconfigured into a steam sterilization mode, as shown in Figs. 14 to 16. Following the removal of the transparent plastic plates from the front port 13 and rear port 14, a first pressure-resistant door 17 is installed at the front port 13, and a second pressure-resistant door 17' is installed at the rear port 14. The first pressure-resistant door 17 at the front port 13 is equipped with a steam inlet/outlet valve 9, and the bottom opening 12 of the valve body 1 has the third pressure-resistant door 18 with the third drainage valve 181. The steam inlet/outlet valve 9 and the drainage valve 181 can be remotely controlled. The second pressure-resistant door 17' at the rear port 14 is equipped with a thermometer 171 and, optionally, a pressure gauge for monitoring the temperature, pressure inside the valve body 1. At this point, the internal transport system of the valve body 1 becomes a pressure vessel, and the equipment and rotary valve in contact with the steam must meet the requirements of the pressure vessel directive. This present invention, in steam sterilization mode, meets the pressure vessel standards. The drainage valve 181 on the third pressure-resistant door 18 has a drainage port for discharging steam condensate. The third pressure-resistant door 18 has a round plate-like structure with a slight taper forming a slope 182 for automatic drainage. Steam is input and output through the steam inlet/outlet valve 9, and the steam condensate is discharged via the drainage valve 181. The steam sterilization process uses saturated steam at a minimum of 130°C (266°F), maintained continuously for at least 0.5 to 2 hours.

After completing the aforementioned cleaning procedures, the reverse assembly can proceed as described earlier. The front door 3 is reattached to cover the front port 13, while the side cover 2, along with the gear motor 5, the motor seat 52, and the transmission shaft 51, is used to push the rotor 6 back into the valve body 1 in a parallel manner. Once the side cover 2 is positioned, the apparatus can return to the transport mode to continue the powdered and granular materials transport process.

In the aforementioned embodiment, the top and bottom openings 11, 12 of the valve body 1 are connected by using a flange structure. In other embodiments, as shown in Figs. 9 to 16, a pipe clamp 19, such as a Tri-Clamp, can be used instead. This facilitates quick disassembly and assembly without the need for additional tools, allowing the lower transport pipeline or the aforementioned drainage valve 18 to be easily removed and reassembled by hand. This enhances the convenience and efficiency of disassembly and assembly in the invention.

While we have shown and described the embodiment in accordance with the present invention, it should be clear to those skilled in the art that further embodiments may be made without departing from the scope of the present invention.

## Claims

1. A hygienic rotary valve, comprising:
a valve body (1) with a top opening (11) and a bottom opening (12), a front port (13) and a rear port (14) defined in a front end and a rear end respectively, guide rails (15) connected to both sides of the valve body (1);
a side cover (2) slidably connected to the guide rails (15), the side cover (2) matching and sealing the rear port (14) of the valve body (1), the side cover (2) including a transmission shaft seat (21);
a transmission shaft (51) extending through the transmission shaft seat (21) and located in the valve body (1), the transmission shaft (51) having a conical section (511) formed at a front end thereof, a recessed section (512) formed in a distal end of the conical section (511), the conical section (511) having a multiple-faced column (513) extending from the distal end of the conical section (511);
a gear motor (5) serving as a direct power source;
a motor seat (52) housing a coupling (53) that connects the gear motor (5) and the transmission shaft (51);
a rotor (6) having a rotor inner ring (61) and multiple blades (63) connected radially to the rotor (6), the rotor inner ring (61) of the rotor (6) having a shaft hole (611) that fits onto the conical section (511) of the transmission shaft (53), the rotor (6) having an inner hexagonal slot (64) located corresponding to the multiple-faced column (513) so that the transmission shaft (53) drives the rotor (6) via the multiple-faced column (513) and the inner hexagonal slot (64);
an end cover (65) engaged with the recessed section (512) of the transmission shaft (51) and covering a connection area between the rotor (6) and the transmission shaft (51);
a front door (3) sealing the front port (13) of the valve body (1), the front door (3) including a first air nozzle (31) located corresponding to the end cover (65), at least one second air nozzle (32) located corresponding to an outer surface of the rotor inner ring (61), the first air nozzle (31) and the second air nozzle (32) connected to a high-pressure air filtration devices (4, 4') respectively, wherein
in a transport mode, the rotor (6) is driven by the power of the gear motor (5) via the transmission shaft (51) so as to transport powdered and granular materials, the high-pressure air filtration devices (4, 4') blow off materials attached to the end cover (65) or a chamber (631) of the rotor (6) through the first air nozzle (31) and the second air nozzle (32);
the valve body (1) being used in a dry cleaning mode, a wet cleaning mode, and a steam sterilization mode, wherein
in the dry cleaning mode, the front door (3) is opened, the gear motor (5) and the side cover (2) are pushed back along the guide rails (15), so that the rotor (6) along with the transmission shaft (51) exit from the rear port (14) of the valve body (1);
in the wet cleaning mode, two cover plates (16) are respectively installed at the front port (13) and rear port (14) after dry cleaning, so that the wet cleaning mode is proceeded through the top opening (11) and bottom opening (12), and
in the steam sterilization mode, a first pressure-resistant door (17) is installed at the front port (13), and a second pressure-resistant door (17') is installed at the rear port (14), a steam inlet/outlet valve (9) is installed via the first pressure-resistant door (17) for high-pressure cleaning.

2. The hygienic rotary valve as claimed in claim 1, wherein, in the wet cleaning mode, the cover plates (16) are transparent, the wet cleaning mode involves at least one of hot water rinsing, detergent spraying, and clean water rinsing through the top opening (11), followed by hot air drying to complete the wet cleaning mode.

3. The hygienic rotary valve as claimed in claim 1, wherein, in the steam sterilization mode, the first pressure-resistant door (17) at the front port (13) of the valve body (1) is equipped with the steam inlet/outlet valve (9), the second pressure-resistant door (17') at the rear port (14) of the valve body (1) has a thermometer (171) and a pressure gauge (172), the bottom opening (12) of the valve body (1) has a third pressure-resistant door (18) with a drainage valve (181) that has a drainage outlet, such that steam is input and output through the steam inlet/outlet valve (9) for steam sterilization.

4. The hygienic grade rotary valve as claimed in claim 1, wherein multiple bearings (22) and at least one shaft seal (25) are located between the transmission shaft seat (21) of the side cover (2) and the transmission shaft (51), the rotor (6) is supported by the bearings (22), the shaft seal (25) prevents intrusion of powdered and granular materials, a plastic sealing ring (23) is installed between the end of the side cover (2) and the conical section (511) of the transmission shaft (51) to block powdered and granular materials from entering a gap between the side cover (2) and the transmission shaft (51), the side cover (2) includes two orifices (24) configured as air injection pipelines which are connected to the high-pressure air filtration devices (4, 4') to introduce high-pressure air to evenly press the plastic sealing ring (23), forcing the plastic sealing ring (23) to seal effectively between the end of the side cover (2) and the conical section (511) of the transmission shaft (51).

5. The hygienic rotary valve as claimed in claim 1, wherein the side cover (2) includes a protruding ring portion (26) corresponding to the rear port (14) of the valve body (1), and an outer circumferential surface of the protruding ring portion(26) has an elastic sealing element (261), which elastically seals between the rear port (14) of the valve body (1) and the side cover (2), the side cover (2) has a recessed annular groove (27) at a position corresponding to the rotor (6), with an elastic shaft seal (271) and a wear-resistant shaft seal (272) sequentially arranged from inside to outside in the annular groove (27), the wear-resistant shaft seal (272) is in contact with the rotor (6).

6. The hygienic rotary valve as claimed in claim 1, wherein the valve body (1) includes multiple legs (121) which protrude from a bottom end of the valve body (1) to support the valve body (1).

7. The hygienic rotary valve as claimed in claim 1, wherein the valve body (1) includes two lugs (131), the front door (3) is mounted onto the lugs (131) via bolts (33) so that the front door (3) of the valve body (1) is rotated to be open and closed.

8. The hygienic rotary valve as claimed in claim 1, further comprising a tool (8) placed on the valve body (1), one of two ends of the tool (8) is a pry bar (81) which has a hook (811) corresponding to the rotor inner ring (61) of the rotor (6), the hook (811) is engaged with the rotor inner ring (61) of the rotor (6) when removing the rotor (6) from the transmission shaft (51), another one of the two ends of the tool (8) is a socket (82) for assembling or disassembling the end cover (65).

9. The hygienic rotary valve as claimed in claim 8, wherein a guard plate (152) is connected to distal ends of the guide rails (15) to position the guide rails (15) in a parallel position with each other, the guard plate (152) has a storage rack (153), and the pry bar (81) is placed on the storage rack (153).

10. The hygienic rotary valve as claimed in claim 1, wherein the front door (3) has at least one viewing window (7), the at least one viewing window (7) is fitted with a transparent panel (71) for observation of an interior of the valve body (1) during the transport mode.
